# EUROPÄISCHE PATENTSCHRIFT

(11) **EP 0 700 414 B1**
(45) Veröffentlichungstag und Bekanntmachung des Hinweises auf die Patenterteilung: **06.08.1997**
(21) Anmeldenummer: 93909536.0
(22) Anmeldetag: 03.05.1993
(51) Int. Cl.: C08L 1/28, C08L 3/00, C08L 5/00, A61L 15/28, A61L 15/60

(54) **POLYMERZUSAMMENSETZUNG, ABSORPTIONSMATERIALZUSAMMENSETZUNG, DEREN HERSTELLUNG UND VERWENDUNG**
POLYMER COMPOSITION, ABSORBENT MATERIAL COMPOSITION, THEIR PRODUCTION AND USE
COMPOSITION POLYMERE, COMPOSITION DE MATERIAU ABSORBANT, LEUR PRODUCTION ET LEUR UTILISATION

(43) Veröffentlichungstag der Anmeldung: 13.03.1996
(73) Patentinhaber: Stockhausen GmbH & Co. KG, 47805 Krefeld (DE)
(72) Erfinder: BRÜGGEMANN, Helmut, 47829 Krefeld (DE); GÜNTHER, Uwe, 75392 Deckenpfronn (DE); KLIMMEK, Helmut, D-4150 Krefeld (DE)
(74) Vertreter: Klöpsch, Gerald, Dr.-Ing. Patentanwalt
(86) Internationale Anmeldenummer: EP9301060
(87) Internationale Veröffentlichungsnummer: WO9425519

(56) Entgegenhaltungen:
- EP-A- 0 405 993
- EP-A- 0 481 225
- EP-A- 0 481 226
- DE-A- 4 206 857

## Beschreibung

Die Erfindung betrifft eine Polymerzusammensetzung und eine Absorptionsmaterialzusammensetzung (Absorptionsmaterialien), die überwiegend auf nachwachsenden Rohstoffen basieren und somit grundsätzlich auch biologisch abbaubar sind. Aufgrund der überwiegend nativen Herkunft enthalten die Absorber keine oder deutlich geringere Mengen an Restmonomeren als Absorber auf Polyacrylatbasis. Die erfindungsgemäßen Absorber besitzen eine vergleichsweise hohe Aufnahmekapazität und- geschwindigkeit, auch unter Druck, für Wasser und wäßrige Lösungen, zeigen keine Neigung zum Gelblocking (Gelblocking: Beim Kontakt mit Wasser verkleben die äußeren Schichten des Absorbers und verhindern ein weiteres Vordringen der Flüssigkeit in den Absorber) und sind mechanisch stabil (bezüglich der Entmischung in die Einzelkomponenten). In gequollenem Zustand separieren sie in einzelne Partikel, sie sind nicht wäßrig und weisen eine sehr hohe Gelstabilität auf. Die Erfindung betrifft ferner ein Verfahren zu ihrer Herstellung und ihre Verwendung als Faser, Film, Pulver oder Granulat zur Absorption von Wasser, wäßrigen Lösungen oder wäßrigen Dispersionen und Körperflüssigkeiten in Hygienemitteln wie Tampons oder Windeln und Tierhygieneartikeln, in chemisch-technischen Produkten wie beispielsweise Verpackungsmaterialien insbesondere für Fleisch und Fisch, in Kulturgefäßen sowie zur Bodenverbesserung und als Kabelummantelungen.

Die meisten der heute verwendeten Absorptionsmaterialien, auch Superabsorber genannt, die in der Lage sind, in kurzer Zeit große Flüssigkeitenmengen (Wasser, Urin) aufzunehmen, stellen in erster Linie schwach vernetzte Polyacrylate dar und basieren somit nicht auf nachwachsenden Rohstoffen und sind vergleichsweise unzureichend bzw. überhaupt nicht biologisch abbaubar.

Im Bestreben, Superabsorber aus nachwachsenden Rohstoffen aufzubauen, wurde wie in DE-C-2612846 beschrieben, Acrylsäure auf Polysaccharide, wie z.B. auf Maisstärke, aufgepfropft. Dabei können allerdings nur geringe Mengen an Polysacchariden (bis max. 25 %) eingesetzt werden, da ansonsten drastische Verschlechterungen der Absorptionseigenschaften erhalten werden.

Genauso können durch die Einarbeitung von Polysacchariden ins Polymerisationsgel von Polyacrylaten, wie in DE-OS'en 40 29 591, 40 29 592 und 40 29 593 beschrieben wird, die Polyacrylate nur bis zu max. 25 % ersetzt werden, ohne eine deutliche Verschlechterung des Aufnahmevermögens und anderer Eigenschaften der resultierenden Superabsorber zu erhalten, auch wenn zusätzlich diverse Hilfsstoffe wie Fasern und z.B. Aluminiumvernetzer zugesetzt werden. Die Polysaccharide werden als Bausteine für die Absorber gesehen, um biologisch abbaubare Einheiten zu erhalten.

DE-C-3132976 beschreibt die Vermischung von Polyacrylsäure mit Polysacchariden in Pulverform und in Lösung, wobei die Hülle der Absorberteilchen der Gemische mit Aluminiumvernetzern wie Al(OH)₂OOCCH₃ * 1/3 H₃BO₃ vernetzt wird. Demnach sind auch mit diesem Verfahren keine Superabsorber darstellbar, die zu über 60 % aus nachwachsenden Rohstoffen bestehen.

In den bislang nach dem heutigen Stand der Technik beschriebenen Verfahren besitzen die Polysaccharide keinen entscheidenen Beitrag als Absorptionskomponente.

Zahlreiche Patentveröffentlichungen wie DE-A-2634539 beschreiben die Herstellung von Carboxymethylcellulose-Absorbern, also von Materialien, die prinzipiell biologisch abbaubar sind, durch eine Vernetzung der Carboxymethylcellulose mit diversen Vernetzern im wäßrigen System. Diese Absorber zeigen allerdings ein starkes Gelblocking.

In der US-A-4 959 341 wird die Herstellung eines auf Carboxymethylcellulose basierenden Absorbers beschrieben, der aus einer Mischung von Carboxymethylcellulose, Cellulosefasern, einer hydrophoben Komponente und Al(OH)₂OOCCH₃ * 1/3 H₃BO₃ als Vernetzer besteht, wobei der Aluminiumvernetzer eine Vernetzung der Carboxymethylcellulose während der Flüssigkeitsaufnahme bewirkt.
Diese Absorber besitzen gute Absorptionseigenschaften, zeigen aber Blockphänomene. Zudem werden diese Absorber durch mechanische Belastungen, wie Absiebung oder Förderung, leicht entmischt, so daß sie nicht mehr als homogenes Produkt vorliegen, was ihre Einsatzmöglichkeiten stark einschränkt.

In der EP-B 0 201 895 wird ebenfalls die Herstellung eines Absorbers, der auf Carboxymethylcellulose basiert, beschrieben. Allerdings wird bei der Herstellung dieser Absorber in einer wäßrigen Lösung gearbeitet, in der die Carboxymethylcellulose nur in geringer Konzentration vorliegt. Weiterhin werden bei der Herstellung größere Mengen an organischen Lösungsmitteln benötigt. Die Herstellung dieser Carboxymethycellulose-Absorber erfordert zudem einen hohen Zeitaufwand. Die Absorber selbst zeigen Blockphänomene und eine niedrige Gelstärke.

Während bei der Entwicklung der Superabsorber zunächst allein das sehr hohe Quellvermögen bei Kontakt mit Flüssigkeit, auch freie Quellkapazität genannt, allein im Vordergrund stand, hat sich später gezeigt, daß es nicht nur auf die Menge der absorbierten Flüssigkeit ankommt, sondern auch auf die Gelfestigkeit. Absorbtionsvermögen oder auch Quellvermögen oder freie Quellkapazität genannt einerseits und Gelfestigkeit bei einem vernetzten Polymer andererseits stellten jedoch gegenläufige Eigenschaften dar, wie bereits aus dem US-PS 3,247,171 (DOW/WALKER) bekannt ist, ferner aus der US-PS Re 32, 649. Das bedeutet, daß Polymere mit besonders hohem Absorptionsvermögen nur eine geringe Festigkeit des gequollenen Gels aufweisen mit der Folge, daß das Gel unter einem angewendeten Druck (z. B. Körperdruck) deformierbar ist und die weitere Flüssigkeitsverteilung und -aufnahme verhindert. Nach der US-PS Re 32,649 soll daher ein ausgewogenes Verhältnis zwischen Absorptionsvermögen (Gelvolumen) und Gelstärke angestrebt werden, damit bei der Verwendung derartiger Superabsorber in einer Windelkonstruktion Flüssigkeitsaufnahme, Flüssigkeitstransport, Trockenheit der Windel und der Haut gewährleistet sind. Dabei kommt es nicht nur darauf an, daß das Polymer Flüssigkeit unter nachfolgender Einwirkung eines Drucks zurückhalten kann, nachdem das Polymer frei quellen konnte, sondern auch darauf, Flüssigkeiten auch gegen einen gleichzeitigen, d. h. während der Flüssigkeitsabsorption, ausgeübten Druck aufzunehmen, wie es unter praktischen Gegebenheiten geschieht, wenn ein Baby oder eine Person auf einem Sanitärartikel sitzt oder liegt oder wenn es, z.B. durch Beinbewegung, zur Entwicklung von Scherkräften kommt. Die spezifische Absorptionseigenschaft wird in der EP-A-0 339 461 als Aufnahme unter Druck ("Absorption Under Load" = "AUL") bezeichnet.

Der vorliegenden Erfindung lag die Aufgabe zugrunde, auf einfachem Wege eine Polymerzusammensetzung und eine Absoprtionsmaterialzusammensetzung (im folgenden kurz Absorber genannt) bereitzustellen, der die zuvor beschriebenen Mängel nicht aufweist und der die folgenden Eigenschaften besitzt:
a) Der Absorber soll überwiegend aus Komponenten nativen Ursprungs bestehen und damit grundsätzlich auch biologisch abbaubar sein.
b) Die Absorber sollen eine hohe mechanische Festigkeit aufweisen, sie dürfen sich beim Sieben oder z.B. bei einer Schneckenförderung nicht in ihre einzelnen Komponenten auftrennen.
c) Die Absorber sollen eine vergleichsweise hohe Aufnahme-geschwindigkeit und -kapazität, auch unter Druck, für Wasser und wässrige Lösungen besitzen.
d) Der Gehalt an Restmonomeren soll deutlich geringer liegen als bei herkömmlichen Absorbern auf Basis von Polyacrylaten.
e) Die Absorber sollen im gequollenen Zustand eine sehr hohe Gelstabilität aufweisen; dabei sollen die Absorberkörner separiert, in einzelnen Partikeln vorliegen.
f) Sie dürfen nicht zum Gelblocking neigen.
g) Die Absorber sollen eine hohe Aufnahmegeschwindigkeit und -kapazität unter Druck für Wasser und wäßrige Lösungen besitzen.
h) Die Absorber sollen vergleichsweise einfach herstellbar sein.

Erfindungsgemäß erfolgt die Lösung dieser Aufgabe durch ein Absorptionsmittel, das im wesentlichen aus fünf Komponenten besteht:
- einer Komponente A, die auf speziellen nachwachsenden Polysaccharid-Rohstoffen basieren,
- einer Komponente B, die aus einem speziellen wasserquellbaren Polymeren besteht
- einem Matrixmaterial,
- einem ionischen oder kovalenten Vernetzer.
- einem Reaktivzusatz,
und gegebenfalls unter Zusatz eines Antiblockingmittels.

Die vorliegende Erfindung betrifft somit eine Polymerzusammensetzung, insbesondere Absorptionsmaterialzusammensetzung, im wesentlichen bestehend aus 70 bis 99,99 Gew.-% eines wasserlöslichen und/oder wasserquellbaren Polysaccharids oder Polysaccharidderivats, das gegebenenfalls durch Vernetzung modifiziert worden ist (Komponente A)
und
0,01 bis 30 Gew.-% eines wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und/oder Copolymere durch eine mindestens bifunktionelle Verbindung vernetzt sind (Komponente B)
sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder covalenten Vernetzers
und
0,1 bis 50 Gew.-%, bezogen auf die polymeren Komponenten A und B, wenigstens eines Reaktivzusatzes zur Verbesserung der Aufnahmekapazität und der Aufnahmegeschwindigkeit der polymeren Zusammensetzung,
0 bis 50 Gew.-%, bezogen auf die polymeren Komponenten A plus B, wenigstens eines Antiblockingmittels auf Basis natürlicher und/oder synthetischer Fasern und/oder Materialien mit großer Oberfläche.

Im weiteren betrifft die vorstehende Erfindung eine wirkstoffenthaltende Depotmaterialzusammensetzung, die vorstehende Zusammensetzung aufweist und die den Wirkstoff verzögert freisetzt und die als Wirkstoff zum Beispiel ein Arzneimittel, ein Pestizid, ein Bakterizid und/oder einen Riechstoff enthält.

Dabei wurde überraschenderweise gefunden, daß ein geringfügiger Zusatz von Komponente B zur Komponente A eine deutliche Verbesserung der Absorptionseigenschaften bewirkt. Da nur geringe Zusätze an Komponente B notwendig sind, liegt damit der Restmonomerengehalt an z. B. Acrylsäure eines solchen Absorbers deutlich geringer als bei Absorbern auf Polyacrylatbasis.

Ferner wurde überraschenderweise gefunden, daß durch den Zusatz eines Feststoffes, der als Matrix für das Absorbersystem fungiert, in Kombination mit dem Polymerabsorptionsmittel, einer Mischung aus den Komponenten A und B, und einem ionischen Vernetzer einem Reaktivzusatz und ggf. eines Antiblokkingmittels, ein Absorptionsmittel herstellbar ist, daß eine hohe Aufnahmegeschwindigkeit und -kapazität für Wasser und wässrige Lösungen sowie eine verbesserte mechanische Festigkeit hinsichtlich der Entmischung der einzelnen trockenen Partikel besitzt. Weiterhin liegen die Gele dieses Absorbersystems separiert in einzelnen Partikeln vor.
Der Reaktivzusatz verbessert überraschenderweise die Absorptionseigenschaften, insbesondere auch unter Druck.

Außerdem zeigen diese Absorber überraschenderweise, in Kombination mit den oben genannten Eigenschaften, eine Gelstärke, die deutlich höher liegt, als die von Absorbern, die auf Polyacrylsäurebasis aufbauen.

Als Komponente A sind wasserlösliche und wasserquellbare Polymere auf der Basis von Polysacchariden und deren Derivate geeignet, wie Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke und Mischungen aus den einzelnen Polysacchariden. Die bevorzugten Polymeren sind die anionischen Derivate von Stärke, Guar und Cellulose, wobei Carboxymethylcellulose ein besonders bevorzugtes Material darstellt.

Die aufgeführten Polymeren der Komponente A können durch eine Vernetzung modifiziert werden, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.
Die Umsetzung der Polymeren kann mit ionischen Vernetzern wie z.B. Calcium-, Aluminium-, Zirkon und Eisen(III)- Verbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Ethylen- und Propylencarbonat, mit Aminen wie Polyoxypropylenamine, mit Epoxiverbindungen wie Ethylenglykoldiglydcidylether, Glykoldi- oder triglycidylether und Epichlorhydrin, mit Säureanhydriden wie Bernsteinsäureanhydrid und Maleinsäureanhydrid, mit Aldehyden und mehrfunktionellen (aktivierten) Olefinen wie Bis-(acrylamido)-essigsäure und Methylenbisacrylamid möglich.

Ebenso kommen natürlich Derivate der genannten Verbindungsklassen in Betracht sowie heterofunktionelle Verbindungen mit verschieden funktionellen Gruppen der oben genannten Verbindungsklassen.

Als Komponente B sind wasserquellbare synthetische Polymere oder Copolymere in erster Linie auf der Basis von (Meth-) Acrylsäure und weiterhin auf der Basis von (Meth-) Acrylnitril, (Meth-)Acrylamid, Vinylacetat, Vinylpyrrolidon, Vinylpyridin, Maleinsäure, Maleinsäureanhydrid, Itakonsäure, Itakonsäureanhydrid, Fumarsäure, Vinylsulfonsäure, 2-Acrylamido-2-methylpropansulfonsäure, sowie die Amide, ihre N-Alkylderivate und N,N'-Dialkylderivate, hydroxylgruppenhaltige Ester und aminogruppenhaltige Ester der polymerisationsfähigen Säuren geeignet. Bevorzugt sind vernetzte, teilweise neutralisierte Polyacrylate.

Es können bis zu 98 %, vorzugsweise 50 - 80 % der Säuregruppen neutralisiert sein.

Die Polymeren können durch einen mindestens bifunktionellen Vernetzer vernetzt sein.

Die Herstellung dieser vorstehenden Polymeren erfolgt nach bekannten Verfahren (DE-C-27 06 135, DE-OS 40 15 085 ). Ein besonders bevorzugtes Material als Komponente B stellen Polyacrylate dar, z.B. die von der Chemischen Fabrik Stockhausen GmbH hergestellten FAVOR^{R}-Typen.

Als Matrix sind Feststoffe geeignet, die vorzugsweise bei Raumtemperatur eine weiche Konsistenz besitzen, wie z. B. Triglycerinmonostearat oder spezielle Wachsester. Ebenso sind hochviskose Flüssigkeiten wie z. B. Rizinusöl geeignet. Vorzugsweise sind als Matrix Polycaprolactone geeignet, wie TONE 0230 und 0240 von Union Carbide, die auch modifiziert sein können, wie z.B. durch eine Umsetzung mit Maleinsäureanhydrid.

Durch die Matrix erhält das Absorbersystem vermutlich durch chemische und/oder physikalische Wechselwirkungen eine höhere mechanische Festigkeit, wodurch die Entmischung der Einzelkomponenten durch Transportvorgänge, wie z.B. mittels einer Förderschnecke, oder durch Absiebvorgänge stark vermindert wird. Dadurch kann ein Absorptionsmittel hergestellt werden, das nicht nur hohe Absorptionswerte besitzt, sondern nach einer Konfektionierung bzw. nach Einarbeitung in seinem Verwendungsbereich als homogeneres und somit wirksameres System vorliegt.
Außerdem bewirkt das Einbetten des Absorptionsmaterials in der Matrix überraschenderweise eine deutliche Reduzierung bzw. eine gänzliche Beseitigung des Gelblocking, somit ist eine hohe Aufnahmegeschwindigkeit im ganzen Absorber gewährleistet. Weiterhin wird durch die Matrix der Vernetzer fest an der Oberfläche der einzelnen Absorberpartikel fixiert.

Die Granulierung von Superabsorberfeinstäuben durch Agglomerierungshilfsmittel wird in den Beispielen der DE-PS 37 41 157 und DE-PS 39 17 646 beschrieben. Die so hergestellten Produkte besitzen eine hohe Aufnahmegeschwindigkeit für Wasser und wäßrige Lösungen. Diese Produkte bestehen jedoch nur aus Polyacrylaten und sind folglich vergleichsweise unzureichend bzw. überhaupt nicht biologisch abbaubar. Die Agglomeriesationsmittel dienen nur zur Granulierung eines Produktes und keinesfalls als Matrixmaterial.

Die Anti-Blocking-Mittel vermindern ebenfalls Gelblocking; sie bewirken also eine schnellere und bessere Flüssigkeitsaufnahme und sorgen dafür, daß die Gele separiert, d.h. in einzelnen Partikeln vorliegen.
Geeignete Anti-Blocking-Mittel sind bekanntermaßen (vgl. DE-C-31 41 098 und DE-C-33 13 344) Fasermaterialien und andere Materialien mit großer Oberfläche.
Die Fasern können natürliche oder synthetische Fasern sein, wie z.B. Woll-, Baumwoll-, Seiden- und Cellulosefasern, bzw. Polyamid-, Polyester-, Polyacrylnitril-, Polyurethanfasern, Fasern von Olefinen und deren Substitutionsprodukten sowie Polyvinylalkoholfasern und deren Derivate. Beipiele für anorganische Materialien sind Bentonite, Zeolithe, Aerosile und Aktivkohlen.

Reaktivzusätze bewirken eine Verbesserung der Aufnahmekapazität oder -geschwindigkeit, insbesondere unter Druck. Geeignete Reaktivzusätze sind solche Stoffe, welche die Konzentration der hydrophilen Gruppen im Absorber erhöhen oder/und eine Modifizierung des Absorbers bewirken oder/und die Fließfähigkeit im Gel erhöhen oder/und die Elektrolytkonzentration der zu absorbierenden Flüssigkeit erniedrigen. Der Reaktivzusatz kann chemisch oder physikalisch mit den übrigen Komponenten verbunden sein. Geeignete Reaktivzusätze sind Tenside, Ionentauscher - insbesondere Kationentauscher -oder Metallsalze bzw. Komplexe die während der Darstellung des Aborbersystems hydrolysieren können; dabei kann der Reaktivzusatz auch ganz oder teilweise das Antiblockingmittel ersetzen.
Ein besonders bevorzugtes Material für die Reaktivzusätze ist Titanylsulfat.

Geeignete Vernetzer sind Verbindungen, welche die oben beschriebenen Polymeren in einen Zustand überführen, in dem die Wasserlöslichkeit reduziert wird, das Saugvermögen verbessert und die Blockphänomene vermindert werden.

Als ionische Vernetzer sind Metallverbindungen geeignet, die mit den funktionellen Gruppen der Polymere in Wechselwirkung treten können. Besonders bevorzugt sind Magnesium-, Calcium-, Aluminium-, Zirkon-, Eisen- und Zinkverbindungen, die eine sehr gute Wasserlöslichkeit besitzen, wie die Salze von Carbonsäuren und anorganischen Säuren.

Bevorzugte Carbonsäuren sind Essigsäure, Milchsäure, Salicylsäure, Propionsäure, Benzoesäure, Fettsäuren, Malonsäure, Bernsteinsäure, Glutarsäure, Adipinsäure, Citronensäure, Weinsäure, Äpfelsäure und Schleimsäure.
Bevorzugte anorganische Anionen sind Chloride, Bromide, Hydrogensulfate, Sulfate, Phosphate, Borate, Nitrate, Hydrogencarbonate und Carbonate.
Weiterhin sind organische Verbindungen geeignet, die mehrwertige Metalle enthalten, wie Acetylacetonate und Alkoholate, wie z.B. Fe(acac)₃, Zr(acac)₄, Ti(OBu)₄ und Zr(o-Prop)₄.

Der wasserlösliche Vernetzer bewirkt eine Vernetzung der Komponenten A und B, sowohl untereinander als auch zwischeneinander, besonders an der Oberfläche, wodurch, wie in DE-PS 31 32 976, DE-OS 26 09 144 und US-A-4 959 341 beschrieben ist, die Absorptionseigenschaften verbessert werden.

Als kovalente Vernetzer sind polyfunktionelle Carbonsäuren, Alkohole, Amine, Epoxiverbindungen, Carbonsäureanhydride und Aldehyde ,sowie deren Derivate, geeignet. Beispiele sind Citronensäure, Schleimsäure, Weinsäure, Äpfelsäure, Malonsäure, Bernsteinsäure, Glutarsäure, Adipinsäure, Polyethylenglykole, Glycerin, Propandiole, Polyoxypropylenamine, Epichlorhydrin, Ethylenglykoldiglycidylether, Glykoldiglycidylether, Bernsteinsäureanhydrid, Maleinsäureanhydrid, Ethylencarbonat und Propylencarbonat.

Ebenso kommen natürliche Derivate der genannten Verbindungen in Betracht sowie heterofunktionelle Verbindungen mit verschiedenen funktionellen Gruppen der oben genannte Verbindungsklassen.

Der Anteil an Komponente A an dem Verhältnis Komponente A zu Komponente B beträgt 70 - 99,99 Gew.%, vorzugsweise 75 - 90 Gew.%. Der Anteil an Komponente B beträgt 0,01 - 30 Gew.% vorzugsweise 10 - 25 Gew.%.

Der Zusatz, auch in geringen Mengen, an Komponente B bewirkt eine starke Verbesserung der Absorptionseigenschaften, vor allem des Saugvermögens. Dadurch kann eine überraschend deutliche Verbesserung der Absorptionseigenschaften gegenüber reinem CMC-Material erzielt werden.

Die Menge an Anti-Blocking-Mittel beträgt zwischen 0,5 und 50 Gew. %, vorzugsweise 5 - 15 Gew. % bezogen auf die Komponenten A und B.

Die Menge an Reaktivzusatz beträgt zwischen 0,1 und 50 Gew.%, vorzugsweise 2 - 10 Gew.%, bezogen auf die Komponenten A und B.

Die Vernetzermenge im Absorber beträgt 0,001 - 10 Gew.%, vorzugsweise 3 - 7 Gew.%, bezogen auf die Komponenten A und B.

Der Zusatz von Matrixmaterial bezogen auf die Komponenten A und B soll zwischen 0,1 - 30 Gew.%, vorzugsweise zwischen 2,5 und 7,5 Gew.% betragen.
Die Matrix verhindert ein Auseinanderfallen des Absorptionsmaterials, wie es bei reinen physikalischen Mischungen wie z.B. US-A-4 952 550 beobachtet wird und verhindert zusätzlich ein Gelblocking.

Die vorzugsweise Herstellung des Absorptionsmaterials wird nachfolgend beschrieben.
Zur Herstellung des erfindungsgemäßen Absorptionsmittels werden die Komponente A und die Komponente B physikalisch in trockener Form bei Raumtemperatur vermischt. Dieses Material wird mit dem Anti-Blocking-Mittel, dem Reaktivzusatz und der Matrix-Komponente vermischt, bis eine homogene Mischung vorliegt. Die Mischung der Komponenten erfolgt in geeigneten Mischern wie Schneckenmischer, Wirbelschichtbettmischer, Scheibenmischer oder Bandmischer.
Die Wärmebehandlung findet bei 25 - 180° C, vorzugsweise bei 100 - 120° C statt. Die Erwärmungsdauer beträgt 5 - 60 Minuten, vorzugsweise 20 - 40 Minuten. Zur Wärmebehandlung des Produktes werden gewöhnliche Trockner oder Heizöfen (z.B. Scheibentrockner, Bandtrockner, Wirbelschichtbetttrockner oder Infrarottrockner) eingesetzt.
Anschließend wird der ionische Vernetzer, vorzugsweise Aluminiumdihydroxyacetat, stabilisiert mit Borsäure, bei Raumtemperatur eingearbeitet, bis eine homogene Mischung entstanden ist. Zur Fixierung des Vernetzers durch die Matrix wird nochmals auf 25 - 180° C, vorzugsweise auf 50 - 80° C, für 5 - 60 Minuten erhitzt, um das Matrixmaterial aufzuschmelzen.

Die Komponenten A und B können vor der Vermischung abgesiebt werden, vorzugsweise im Bereich von 90 - 630 µm.

Die Einarbeitung der Matrixkomponenten erfolgt vorzugsweise bei Raumtemperatur, die Matrixkomponente kann aber auch als Schmelze eingesetzt werden.
Die Mischung kann vor der thermischen Modifizierung mit einem vorzugsweise Wasser/Isopropanol-Gemisch versetzt werden, um einen Lösungsvermittler zu haben, der eine thermische Modifizierung der Komponente A, also einem Polysaccharid und nicht von Polyacrylsäure, untereinander, wie auch mit der Matrix-komponente und der Komponente B in den Randbereichen der Komponente A bewirkt, was sich positiv auf das Saugvermögen des Absorptionsmaterials auswirkt. Statt dem Wasser/Isopropanol-Gemisch können auch Wasser und andere Gemische von Wasser mit wasserlöslichen organischen Lösungsmitteln eingesetzt werden. In der EP-PS 0 083 022 wird die Vernetzung eines Absorbers beschrieben, der aus Polyacrylsäure besteht, mit Vernetzern, die wenigstens zwei funktionelle Gruppen enthalten und in der Lage sind, mit den Carboxylgruppen des Polyacrylats zu reagieren. Die Reaktion erfolgt an der Oberfläche der Absorberteilchen. DE-PS 33 14 019 und DE-PS 35 23 617 beschreiben ebenfalls die Oberflächenvernetzung von Polyacrylaten mit Hilfe von Vernetzern, die wenigstens zwei funktionelle Gruppen besitzen. Im Gegensatz zu den erfindungsgemäßen Absorbern werden in diesen Patenten nur Modifizierungen von Polyacrylaten, jedoch nicht von Polysacchariden, in der Hülle beschrieben, wodurch aber keinsfalls Absorber erhalten werden, die ausreichend biologisch abbaubar sind.

Die Einarbeitung des ionischen Vernetzers kann auch direkt in die physikalische Mischung Komponente A, Komponente B, Anti-Blocking-Mittel, Reaktivzusatz und Matrix-Material erfolgen, worauf dann auf 25 - 180° C, vorzugsweise auf 100 - 120° C, für 5 - 120 Minuten, idealerweise für 20 - 60 Minuten erwärmt wird.

Der oben beschriebene Lösungsmittel-Schritt kann bei diesem Verfahren vor oder nach der Vernetzereinarbeitung erfolgen.

Der kovalente Vernetzer kann alternativ und zusätzlich zu dem ionischen Vernetzer zu der Polymermischung vor oder nach der Matrixzugabe zugesetzt werden. Der kovalente Vernetzer wird in einem vorzugsweise gegebenfalls Alkohol/Wasser-Gemisch gelöst und der Polymermischung unter schnellem Rühren zugetropft. Die Lösungsmittel-Menge beträgt zwischen 1 und 10 % bezogen auf die Polymermischung. Anschließend wird auf 25 - 180°C für 5 - 120 Minuten erwärmt. Als Lösungsmittel können Wasser und Gemische aus Wasser mit wasserlöslichen organischen Lösungsmitteln verwendet werden.

Das erfindungsgemäße Absorptionsmaterial zeigt eine gute biologische Abbaubarkeit gegenüber Produkten, die auf einer Polyacrylsäurebasis beruhen, bei einem, gegenüber bisher bekannten Absorptionsmaterialien auf nativer Basis stark verbesserten Aufnahme- und Saugvermögen für eine 0,9 %ige Kochsalzlösung, auch unter Druck, bei einer überraschend sehr hohen Gelstärke.

| Gelstärke einiger erfindungsgemäßer Absorber sowie einiger markt bekannter Absorber | |
|---|---|
| Produktbezeichnung | Gelstärke (10 Hz) (N/m²) |
| erfindungsgemäße Absorber | |
| Superabsorber aus Bsp. 4 | ≥ 10000 |
| Superabsorber aus Bsp. 8 | = 10000 |
| marktbekannte Absorber | |
| Produkt A | 2450 |
| Produkt B | 4200 |
| Produkt C | 3500 |
| Produkt D | 2700 |
| Produkt E | 4950 |
| Produkt F | 3700 |
| Produkt G | 1575 |

Produkte A, B, C, D, F, G:
vernetzte, teilweise neutralisierte Polyacrylate
Produkt E:
vernetztes, teilweise neutralisiertes Polyacrylat-Stärke-Pfropfpolymer
Weiterhin ist die mechanische Festigkeit (in Bezug auf Entmischung in die Einzelkomponenten) gegenüber den zuvor beschriebenen, auf nachwachsenden Rohstoffen basierenden Absorbern deutlich verbessert.

Die erfindungsgemäße Polymerzusammensetzung kann insbesondere als Absorptionsmaterial als Faser, Film, Pulver oder Granulat eingesetzt werden, um Wasser oder wässrige Flüssigkeiten, wie Urin und Blut, aufzunehmen und ist somit besonders für den Einsatz in Windeln, Tampons, chirurgischen Erzeugnissen, Kabelummantelungen, Kulturgefäßen, Verpackungsmaterialien für Fleisch oder Fisch und absorbierenden Kleidungsstücken geeignet.

Außerdem ist das Material als Speichermedium zur sukzessiven Freisetzung von Wirkstoffen, wie Arzneimitteln, Pestiziden (US 4,818,534; US 4,983,389; US 4,983,390; US 4,985,251) und Riechstoffen geeignet, mit dem Vorteil, daß das Speichermedium abbaubar ist.
Dadurch ergibt sich als weiterer Vorteil, daß der Wirkstoff vollständig freigesetzt wird.

Die wirkstoffhaltigen Depotmaterialien können durch Absorption, vorzugsweise konzentrierter, wässriger oder wasserhaltiger Lösungen in den weitgehend trockenen Absorber und ggf. dessen erneute Trocknung hergestellt werden.

Der Wirkstoff kann auch direkt oder als Lösung oder Dispersion bei beliebigen Vorstufen des Herstellungsprozesses der Absorberzusammensetzung zugesetzt werden.

Die wirkstoffhaltigen Depotmaterialien werden in Pulverform oder als Dispersion in hydrophoben Medien, die dispersionsstabilisierende Mittel wie Emulgatoren oder Stabilisatoren enthalten können, oder in Mischung mit anderen Stoffen wie Polysacchariden verwendet.

Beispielsweise wird durch den Zusatz solcher bakterizidhaltiger Depotmaterialien zu Cellulose-, Guar- oder Stärkeprodukten oder deren Derivate, wie Carboxymethylcellulose, bei deren Lagerung und Anwendung in wässrigen Medien der Abbau dieser Substanzen über längere Zeit verhindert und wobei durch die Depotwirkung größere Mengen von freiem Wirkstoff in der Lösung vermieden werden können.

### Testmethoden:

### Teebeutel-Test (TBT)

Zur Bestimmung des Absorptionsvermögens wurde ein Teebeutel-Test durchgeführt. Als Prüflösung wurde eine wäßrige 0,9 %ige NaCl-Lösung verwendet.
0,2 g einer Prüfsubstanz (abgesiebt zwischen 90 und 630 µm), die in einem Teebeutel eingewogen wurde, ließ man 10 bzw. 30 Minuten in der Prüflösung aufquellen. Nach fünfminütigem Abtropfen (Maximalwert) wurde in einer Zentrifuge, wie z.B. in einer handelsüblichen Wäscheschleuder, bei 1400 Upm abgeschleudert. Die Flüssigkeitsaufnahme wurde gravimetrisch ermittelt und auf 1 g Substanz umgerechnet. (Retentionswert).

### Absorption under Load (AUL)

Um das Flüssigkeitsaufnahmevermögen unter Druck zu bestimmen, wurde die Absorption under Load , wie in EP-A-0 339 461 beschrieben, bestimmt.
0,16 g Prüfsubstanz (abgesiebt zwischen 300 und 600 µm) ließ man durch Kapillarwirkung für 60 Minuten unter einem Druck von 1,55 kN/m» 99,8 g/in²) in 0,9 %ige NaCl-Lösung quellen. Die Flüssigkeitsaufnahme wurde gravimetrisch ermittelt und auf 1 g Substanz umgerechnet.

### Gelstärke (G')

Um die Gelstärke G' der gequollenen Absorber zu bestimmen, wurde, wie in EP-A-0 339 461 beschrieben, vorgegangen. Gerät: Controlled Stress Rheometer CS 100, (Carri-Med Ltd. Dorking/UK).
Meßbedingungen: Plate-Platte-System, Durchmesser 60 mm, Plattenabstand 2 mm, Temperatur 20°C, Drehmoment 1000 - 4000 µNm, Amplitude 1,5 - 5 mrad, Frequenz 10,0 Hz, 28 ml 0,9 % NaCl/g Absorber. Die Angaben erfolgen in N/m².

### Fließtest (FT)

Mittels des Fließtests wurde ermittelt, wie schnell die Produkte die Test flüssigkeit aufnahmen, ob sie Blockphänomene zeigten, vollständig durchgequollen waren und überall benetzt waren. Weiterhin wurde untersucht, ob die Gele fest, klebrig oder locker und separiert vorlagen.

Zur Durchführung des Fließtests wurden ca. 100 mg Substanz auf ein mit Wasser getränktes Papiertuch gegeben und verfolgt, wie das Wasser von den Produkten aufgesaugt wurde. Das Absorptionsverhalten wurde nach folgender Notenskala bewertet.
- A:: zieht schnell an
- B:: zieht sehr schnell an
- C:: zieht von Anfang bis Ende durch
- D:: Gel liegt nach der Wasseraufnahme se periert vor
- E:: Gelblocking

### Beispiel 1

8 g CMC Walocel 40 000 (Natriumcarboxymethylcellulose, Wolff Walsrode) wird mit 2 g Favor^{R} SAB 953 (vernetztes, teilweise neutralisiertes Natriumpolyacrylat der Fa. Stockhausen GmbH), 0,5 g TONE 230 (Polyol auf der Basis von Caprolactan, Molgewicht 1250 g/Mol, Union Carbide), 0,1 g Titanylsulfat und 0,5 g Al(OH)₂OOCCH₃*1/3 H₃BO₃ unter Verwendung von 2 ml Isopropanol und 1 ml Wasser kräftig gemischt und für 60 Minuten auf 120°C im Ofen erhitzt.
TBT (max./ret.) = 51 g/g / 33 g/g; AUL = 15,4 g/g; FT: B C D

### Beispiel 2

Es wird wie in Beispiel 1 verfahren, jedoch wird die zugesetzte Menge an Titanylsulfat auf 0,25 g erhöht.
TBT (max./ret.) = 47 g/g / 29 g/g; AUL = 17,5 g/g; FT: B C D

### Beispiel 3

Es wird wie in Beispiel 1 verfahren, jedoch wird die zugesetzte Menge an Titanylsulfat auf 0,5 g erhöht.
TBT (max./ret.) = 46 g/g / 28 g/g; AUL = 17,7 g/g; FT: B C D

### Beispiel 4

Es wird wie in Beispiel 2 verfahren, jedoch werden zusätzlich 0,5 g der Faser BE 600/30 (Cellulose, Durchmesser 17 mm, Länge 30 mm, Fa. Rettenmaier) eingearbeitet.
TBT (max./ret.) = 48 g/g / 32 g/g; AUL = 17,4 g/g; FT: A C D

### Beispiel 5

60 g CMC, Walocal 40000 werden mit 1,5 ml Ethylencarbonat, 1,5 ml Isopropanol und 1,5 ml Wasser kräftig gemischt und anschließend für eine Stunde auf 120°C im Ofen erhitzt. 8 g dieses Produktes werden mit 2 g Favor SAB 953, 0,5 g TONE 230, 0,5 g Faser BE 600/30, 0,25 g Titanylsulfat, 1 ml wasser und 2 ml i-Propanol kräftig gemischt und anschließend für eine Stunde auf 120°C im Ofen erhitzt.
TBT (max./ret.) = 46 g/g / 28 g/g; AUL = 18,2 g/g; FT: B C D

### Beispiel 6

Es wir wie in Beispiel 5 verfahren, jedoch werden die statt 0,5 g Faser 1,0 g dieser Faser eingesetzt.
TBT (max./ret.) = 43 g/g / 26 g/g; AUL = 17,5 g/g; FT: B C

### Beispiel 7

Es wird wie in Beispiel 3 verfahren, jedoch werden zusätzlich 0,5 g Faser BE 600/30 eingesetzt. Außerdem wird statt des TONE 230 1,0 g Triglycerinmonostearat eingesetzt.
TBT (max./ret.) = 35 g/g / 24 g/g; AUL = 17,8 g/g; FT: C D

### Beispiel 8

Es wird wie in Beispiel 4 verfahren, jedoch werden 1,0 g der Faser BE 600/30 zugesetzt, außerdem wird Mischung die statt für eine Stunde 1,5 Stunden auf 120°C im Ofen erhitzt.
TBT (max./ret.) = 47 g/g / 31 g/g; AUL = 19,0 g/g; FT: A C D

### Beispiel 9

100 g des in Beispiel 1 erhaltenen Produktes werden mit 100 ml einer 0,125 %igen wässrigen Lösung von 3,7-Bis(dimethylamino)-phenothiaziniumchlorid gemischt und anschließend für 2 h im Umlufttrockenschrank bei 60° C getrocknet.
200 mg des so erhaltenen Produktes werden in einen Teebeutel gegeben. Dieser wird in ein Becherglas mit 50 ml 0,2 %iger Kochsalzlösung eingehängt. Nach einer Stunde wird der Teebeutel entfernt. Die Färbung der Kochsalzlösung wird beurteilt, danach wird der Vorgang mit neuer NaCl-Lösung wiederholt. Auch nach dem 5. Zyklus zeigt die Blaufärbung der Kochsalzlösung die Freisetzung des Wirkstoffs aus der als Speichermedium fungierenden Polymerzusammensetzung an.

### Vergleichsbeispiel 1

Es wird wie in Beispiel 3 verfahren, jedoch werden statt des Titanylsulfats 0,5 g Titandioxid eingesetzt.
TBT (max./ret.) = 47 g/g / 32 g/g; AUL = 10,6 g/g; FT: E

### Vergleichsbeispiele 2,3

Die Beispiele 3 und 5 werden ohne den Zusatz von TONE 230 wiederholt. Die Produkte sind inhomogen und entmischen sich leicht. Reproduzierbare Analysedaten können daher nicht erhalten werden.

### Vergleichsbeispiel 4

Es wird wie in Beispiel 4 verfahren, jedoch wird kein Titanylsulfat zugesetzt.
TBT (max./ret.) = 45 g/g / 33 g/g; AUL = 9,9 g/g; FT: A C D

## Patentansprüche

1. Polymerzusammensetzung, insbesondere Absorptionsmaterialzusammensetzung, im wesentlichen bestehend aus 70 bis 99,99 Gew.-% eines wasserlöslichen und/oder wasserquellbaren Polysaccharids oder Polysaccharidderivats, das gegebenenfalls durch Vernetzung modifiziert worden ist (Komponente A)
und
0,01 bis 30 Gew.-% eines wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und/oder Copolymere durch eine mindestens bifunktionelle Verbindung vernetzt sind (Komponente B)
sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder covalenten Vernetzers
und
0,1 bis 50 Gew.-%, bezogen auf die polymeren Komponenten A und B, wenigstens eines Reaktivzusatzes zur Verbesserung der Aufnahmekapazität und der Aufnahmegeschwindigkeit der polymeren Zusammensetzung,
0 bis 50 Gew.-%, bezogen auf die polymeren Komponenten A plus B, wenigstens eines Antiblockingmittels auf Basis natürlicher und/oder synthetischer Fasern und/oder Materialien mit großer Oberfläche.

2. Wirkstoffenthaltende Zusammensetzung, im wesentlichen bestehend aus einer Zusammensetzung nach Anspruch 1 sowie wenigstens einem Wirkstoff, beispielsweise einem Arzneimittel, einem Pestizid, einem Bakterizid und/oder einem Riechstoff.

3. Zusammensetzung nach Ansprüchen 1 bis 2, im wesentlichen bestehend aus
75 - 90 Gew.-% der Komponente A,
10 - 25 Gew.-% der Komponente B,
als polymere Komponenten und
2,5 - 7,5 Gew.-%, bezogen auf die polymeren Komponenten A und B, wenigstens eines Matrixmaterials,
3 - 7 Gew.-%, bezogen auf die polymeren Komponenten A und B, wenigstens eines Vernetzers,
2 - 10 Gew.-%, bezogen auf die polymeren Komponenten A und B, wenigstens eines Reaktivzusatzes und
0,5 - 50 Gew.-%, vorzugsweise 0,5 - 15 Gew.-%, bezogen auf die polymeren Komponenten A und B, wenigstens eines Antiblockingmittels.

4. Zusammensetzung nach Ansprüchen 1 bis 3, dadurch gekennzeichnet, daß die Komponente A ein wasserlösliches und/oder wasserquellbares Polymer auf Basis von Polysacchariden und deren Derivaten, insbesondere Stärke-, Guar- oder Cellulosederivaten ist.

5. Zusammensetzung nach Anspruch 4, dadurch gekennzeichnet, daß Komponente A ein anionisches Derivat von Cellulose, insbesondere Carboxymethylcellulose ist.

6. Zusammensetzung nach Ansprüchen 1 bis 3, dadurch gekennzeichnet, daß das Matrixmaterial bei Raumtemperatur eine hochviskose flüssige oder wachsartige weiche Konsistenz besitzt.

7. Zusammensetzung nach Anspruch 6, dadurch gekennzeichnet, daß das Matrixmaterial ausgewählt ist aus Triglycerinmonostearat, Rizinusöl, speziellen Wachsestern und Polycaprolactonen, die gegebenenfalls durch Umsetzung mit Maleinsäureanhydrid modifiziert sind.

8. Zusammensetzung Ansprüchen 1 bis 3, dadurch gekennzeichnet, daß die ionischen Vernetzer ausgewählt sind aus Metallverbindungen, vorzugsweise wasserlöslichem Magnesium-, Calzium-, Aluminium-, Zirkon-, Eisen- und Zinkverbindungen in Form ihrer Salze mit organischen und anorganischen Säuren.

9. Zusammensetzung nach Ansprüchen 1 bis 3, dadurch gekennzeichnet, daß die kovalenten Vernetzer ausgewählt sind aus polyfunktionären Carbonsäuren, Alkoholen, Aminen, Epoxiden, Carbonsäureanhydriden und/oder Aldehyden sowie deren Derivaten und deren heterofunktionelle Verbindungen mit verschiedenen funktionellen Gruppen der genannten Verbindungsklassen.

10. Zusammensetzung nach Ansprüchen 1 bis 3, dadurch gekennzeichnet, daß der Reaktivzusatz ausgewählt ist aus natürlichen und/oder synthetischen Tensiden, Ionenaustauschern, insbesondere Kationaustauscher und/oder hydrolysierbaren Metallsalzen oder Metallsalzkomplexen.

11. Zusammensetzung nach Anspruch 10, dadurch gekennzeichnet, daß der Reaktivzusatz Titanylsulfat ist.

12. Verfahren zur Herstellung einer Zusammensetzung nach Ansprüchen 1 bis 11, dadurch gekennzeichnet, daß die Komponenten zunächst miteinander bis zur Homogenität vermischt und dann der Vernetzer durch die Matrix mittels einer abschließenden Wärmebehandlung fixiert wird.

13. Verfahren nach Anspruch 12, dadurch gekennzeichnet, daß man die Komponenten A und B vor dem Vermischen auf eine Korngröße von 90 µm - 630 µm absiebt.

14. Verfahren nach Anspruch 12 oder 13, dadurch gekennzeichnet, daß man zunächst die Komponenten A und B vermischt, das Antiblockingmittel, den Reaktivzusatz und das Matrixmaterial bis zum Erreichen der Homogenität einmischt, indem man diese Mischung einer Wärmebehandlung bei 25 °C bis 180 °C, vorzugsweise 100 °C bis 120 °C, unterzieht und nach der Zugabe des Vernetzers diesen auf der Matrix bei 25 °C bis 180 °C, vorzugsweise 50 °C bis 80 °C, fixiert.

15. Verfahren nach Anspruch 12 oder 13, dadurch gekennzeichnet, daß man alle Komponenten zunächst physikalisch mischt und dann eine Wärmebehandlung bei 25 °C bis 180 °C, vorzugsweise 100 °C bis 120 °C, durchführt um den Vernetzer auf der Matrix zu fixieren.

16. Verfahren nach Ansprüchen 12 bis 15, dadurch gekennzeichnet, daß man die Komponenten vor der abschließenden Wärmebehandlung mit einem hydrophilen Lösungsmittel, vorzugsweise in Mengen von 1 - 10 Gew.-%, bezogen auf die Polymermischung, versetzt.

17. Verfahren nach Anspruch 16, dadurch gekennzeichent, daß das Lösemittel Wasser oder ein Gemisch von Wasser mit einem hydrophilen organischen Lösemittel, bevorzugt ein Wasser/-Isopropanol-Gemisch ist.

18. Verfahren zur Herstellung einer Depotmaterialzusammensetzung nach Anspruch 2, dadurch gekennzeichnet, daß der Wirkstoff entweder aus wäßrigen oder wasserenthaltenden Lösungen von der Zusammenstzung nach Ansprüchen 1, 3 bis 11 absorbiert wird und gegebenenfalls erneut getrocknet wird oder als Lösung oder Dispersion bei beliebigen Vorstufen des Herstellungsverfahrens der besagten Zusammensetzung zugesetzt wird.

19. Verwendung der Zusammensetzung nach Ansprüchen 1 und 3 bis 11 oder hergestellt nach Ansprüchen 12 bis 18 aus Faser, Film, Pulver oder Granulat zur Absorption von wasserenthaltenden Lösungen und Dispersionen oder (Körper-)Flüssigkeiten, wie zum Beispiel Urin oder Blut, in chemisch-technischen Produkten wie beispielsweise Verpackungsmaterialien, Kulturgefäßen, zur Bodenverbesserung und als Kabelummantelung, in Hygieneartikeln wie beispielsweise Tampons oder Windeln oder Tierhygienemitteln.

20. Verwendung der Zusammensetzung nach Ansprüchen 2 bis 11 oder hergestellt nach Anspruch 18, in Pulverform oder als Dispersion in hydrophoben Medien, gegebenenfalls in Kombination mit Dispersionsstabilisatoren oder in Mischung mit anderen Stoffen.

21. (Tier) Hygieneartikel, enthaltend eine Zusammensetzung nach Ansprüchen 1 bis 11 oder hergestellt nach Ansprüchen 12 bis 18.

22. Chemisch-technische Produkte, enthaltend die Zusammensetzung nach Ansprüchen 1 bis 11 oder hergestellt nach Ansprüchen 12 bis 18.

## Claims

1. A polymer composition, in particular absorbent composition, substantially consisting of 70 to 99.99%-wt. of a water-soluble and/or water-swellable polysaccharide or polysaccharide derivative which has optionally been modified by cross-linkage (component A)
and
0.01 to 30%-wt. of a water-swellable, synthetic polymer and/or copolymer of (meth)acrylic acid, (meth)acrylonitrile, (meth)acrylamide, vinyl acetate, vinyl pyrrolidone, vinyl pyridine, maleic acid (anhydride), itaconic acid (anhydride), fumaric acid, vinyl sulfonic acid, and/or 2-acrylamido-2-methylpropane sulfonic acid, the amides, the N-alkyl derivatives, the N,N'-dialkyl derivatives, the hydoxyl group-containing esters and amino group-containing esters of these polymerizable acids, with 0 to 98%-wt. of the acid groups of these acids being neutralized, and these polymers and/or copolymers being cross-linked by an at least bifunctional compound (component B)
and
0.1 to 30%-wt., relative to the polymer components A and B, of an organic matrix material having a melting or softening point of below 180°C to prevent separation and gel blocking,
0.001 to 10%-wt., relative to the two polymer components A and B, of an ionic and/or covalent cross-linking agent,
and
0.1 to 50%-wt., relative to the polymer components A and B, of at least one reactive additive to improve the absorption capacity and the absorption rate of the polymer composition,
0 to 50%-wt., relative to the polymer components A plus B, of at least one anti-blocking agent based on natural and/or synthetic fibers and/or large-surface materials.

2. An active substance-containing composition substantially consisting of a composition according to claim 1 and at least one active substance, for example, a drug, a pesticide, a bactericide, and/or a perfume.

3. The composition according to claim 1 or 2 substantially consisting of
75 - 90%-wt. of component A,
10 - 25%-wt. of component B
as polymer components, and
2.5 - 7.5%-wt., relative to the polymer components A and B, of at least one matrix material,
3 - 7%-wt., relative to the polymer components A and B, of at least one cross-linking agent,
2 - 10%-wt., relative to the polymer components A and B, of at least one reactive additive, and
0.5 - 50%-wt., preferably 0.5 - 15%-wt., relative to the polymer components A and B, of at least one anti-blocking agent.

4. The composition according to claims 1 to 3 characterized in that component A is a water-soluble and/or water-swellable polymer based on polysaccharides and their derivatives, in particular starch, guar, or cellulose derivatives.

5. The composition according to claim 4 characterized in that component A is an anionic derivative of cellulose, in particular is carboxymethylcellulose.

6. The composition according to claims 1 to 3 characterized in that the matrix material has a highly viscous liquid or waxy soft consistency at room temperature.

7. The composition according to claim 6 characterized in that the matrix material is selected from triglycerol monostearate, castor oil, special wax esters, and polycaprolactones, which are optionally modified by reaction with maleic anhydride.

8. The composition according to claims 1 to 3 characterized in that the ionic cross-linking agents are selected from metallic compounds, preferably water-soluble magnesium, calcium, aluminum, zirconium, iron, and zinc compounds, in the form of their salts with organic and inorganic acids.

9. The composition according to claims 1 to 3 characterized in that the covalent cross-linking agents are selected from polyfunctional carboxylic acids, alcohols, amines, epoxides, carboxylic acid anhydrides, and/or aldehydes, as well as their derivatives and their heterofunctional compounds with different functional groups of the mentioned compound classes.

10. The composition according to claims 1 to 3 characterized in that the reactive additive is selected from natural and/or synthetic surfactants, ion exchangers, in particular cation exchangers and/or hydrolyzable metallic salts or metal salt complexes.

11. The composition according to claim 10 characterized in that the reactive additive is titanyl sulfate.

12. A process for the production of a composition according to claims 1 to 11, characterized in that the components are mixed with one another up to homogeneity first, and that the cross-linking agent is then fixed by the matrix by means of a final heat treatment.

13. The process according to claim 12 characterized in that the components A and B are screened to a particle size of 90 µm - 630 µm, prior to mixing.

14. The process according to claim 12 or 13 characterized in that the components A and B are mixed first, that the anti-blocking agent, the reactive additive and the matrix material are mixed thereto until homogeneity is reached by means of subjecting said mixture to a heat treatment at 25°C to 180°C, preferably 100°C to 120°C, and that, after addition of the cross-linking agent, the same is fixed on the matrix at 25°C to 180°C, preferably 50°C to 80°C.

15. The process according to claim 12 or 13 characterized in that all of the components are mixed physically first, and that then a heat treatment at 25°C to 180°C, preferably 100°C to 120°C, is conducted to fix the cross-linking agent on the matrix.

16. The process according to claims 12 to 15 characterized in that a hydrophilic solvent, preferably in amounts of 1 - 10%-wt., relative to the polymer mixture, is added to the components prior to the final heat treatment.

17. The process according to claim 16 characterized in that the solvent is water or a mixture of water with a hydrophilic organic solvent, preferably is a water/isopropanol mixture.

18. A process for the production of a depot material composition according to claim 2, characterized in that the active substance is either absorbed by the composition according to claims 1, 3 to 11 from aqueous or hydrous solutions and is optionally dried again, or added to said composition as a solution or dispersion in any of the initial stages of the production process.

19. The use of the composition according to claims 1 and 3 to 11 or manufactured according to claims 12 to 18 as fiber, film, powder, or granular material for the absorption of hydrous solutions and dispersions, or (body) fluids, such as urine or blood, in chemico-technical products, such as packaging materials, in culture pots, for soil conditioning and as cable sheathing, in hygiene products, such as tampons or diapers or products for animal hygiene.

20. The use of the composition according to claims 2 to 11 or manufactured according to claim 18 in the form of a powder or as a dispersion in hydrophobic media, optionally in combination with dispersion stabilizers or in admixture with other substances.

21. (Animal) Hygiene items comprising a composition according to claims 1 to 11 or manufactured according to claims 12 to 18.

22. Chemico-technical products comprising the composition according to claims 1 to 11 or manufactured according to claims 12 to 18.

## Revendications

1. Composition de polymère, plus particulièrement, composition de matière absorbante, qui se compose, en essence, de 70 à 99,9% en poids d'un polysaccharide ou d'un dérivé d'un polysaccharide, soluble dans l'eau et/ou gonflable à l'eau, qui a éventuellement été modifié par réticulation (composant A)
et
0,01 à 30% en poids d'un polymère et/ou d'un copolymère synthétique et gonflable à l'eau de l'acide (méth)acrylique, du (méth)acrylonitrile, du (méth)acrylamide, de l'acétate de vinyle, de la vinylpyrrolidone, de la vinylpyridine, de l'acide (anhydride) maléique, de l'acide (anhydride) itaconique, de l'acide fumarique, de l'acide vinylsulfonique et/ou de l'acide 2-acrylamido-2-méthylpropanesulfonique, des amides, des dérivés N-alkyliques, des dérivés N,N'-dialkyliques, des esters contenant des radicaux hydroxyle et des esters contenant des radicaux amino de ces acides polymérisables, où de 0 à 98% en poids des radicaux acide de ces acides sont neutralisés et où ces polymères et/ou copolymères sont réticulés par au moins un composé bifonctionnel (composant B),
comme aussi
0,1 à 30% en poids, par rapport aux composants polymériques A et B, d'une matière formant matrice, possédant un point de fusion ou de ramollissement inférieur à 180°C, en vue d'empêcher la démixtion et le blocage du gel
0,001 à 10% en poids, par rapport aux deux composants polymériques A et B, d'un agent de réticulation ionique et/ou covalent
et
0,1 à 50% en poids, par rapport aux composants polymériques A et B, d'au moins un additif réactif destiné à l'amélioration du pouvoir d'absorption et de la vitesse d'absorption de la composition polymérique,
0 à 50% en poids, par rapport aux composants polymériques A plus B, d'au moins un agent antiblocage, à base de fibres naturelles et/ou synthétiques et/ou de matières à grande surface.

2. Composition contenant des principes actifs, constituée, en essence, d'une composition suivant la revendication 1, comme aussi d'au moins un principe actif, par exemple, un médicament, un pesticide, un bactéricide et/ou un parfum.

3. Composition suivant l'une quelconque des revendications 1 et 2, constituée, en essence, de
75 à 90% en poids du composant A,
10 à 25% en poids du composant B,
à titre de composants polymériques et
2,5 à 7,5% en poids, par rapport aux composants polymériques A et B, d'au moins une matière formant matrice,
3 à 7% en poids, par rapport aux composants polymériques A et B, d'au moins un agent de réticulation,
2 à 10% en poids, par rapport aux composants polymériques A et B, d'au moins un additif réactif et
0,5 à 50% en poids, de préférence, 0,5 à 15% en poids, par rapport aux composants polymériques A et B, d'au moins un agent antiblocage.

4. Composition suivant les revendications 1 à 3, caractérisée en ce que le composant A est un polymère soluble dans l'eau et/ou gonflable à l'eau, à base de polysaccharides et de leurs dérivés, plus particulièrement des dérivés d'amidon, de gomme guar ou de cellulose.

5. Composition suivant la revendication 4, caractérisée en ce que le composant A est un dérivé anionique de la cellulose, plus particulièrement, la carboxyméthylcellulose.

6. Composition suivant les revendications 1 à 3, caractérisée en ce que la matière formant matrice possède, à la température ambiante, une consistance fluide extrêmement visqueuse ou molle cireuse.

7. Composition suivant la revendication 6, caractérisée en ce que l'on choisit la matière formant matrice parmi le monostéarate de triglycérine, l'huile de ricin, des esters cireux particuliers et des polycaprolactones, qui ont éventuellement subi une modification par réaction avec l'anhydride de l'acide maléique.

8. Composition suivant les revendications 1 à 3, caractérisée en ce que l'on choisit l'agent de réticulation ionique parmi des composés de métaux, de préférence, des composés du magnésium, du calcium, de l'aluminium, du zirconium, du fer et du zinc, solubles dans l'eau, sous forme de leurs sels avec des acides organiques et inorganiques.

9. Composition suivant les revendications 1 à 3, caractérisée en ce que l'on choisit l'agent de réticulation covalent parmi des aldéhydes, des anhydrides d'acides carboxyliques, des époxydes, des amines, des alcools et/ou des acides carboxyliques, polyfonctionnels, ainsi que leurs dérivés et leurs composés hétérofontionnels avec divers radicaux fonctionnels des classes de composés citées.

10. Composition suivant les revendications 1 à 3, caractérisée en ce que l'on choisit l'additif réactif parmi des tensioactifs ou surfactifs naturels et/ou synthétiques, des échangeurs d'ions, plus particulièrement des échangeurs de cations et/ou des sels de métaux ou des complexes de sels de métaux hydrolysables.

11. Composition suivant la revendication 10, caractérisée en ce que l'additif réactif est le sulfate de titanyle.

12. Procédé de préparation d'une composition suivant les revendications 1 à 11, caractérisée en ce que l'on mélange d'abord les composants mutuellement jusqu'à parvenir à l'homogénéité et on fixe ensuite l'agent de réticulation à travers la matrice à l'aide d'un traitement thermique final.

13. Procédé suivant la revendication 12, caractérisé en ce que l'on tamise ou crible les composants A et B avant le mélange, jusqu'à une granulométrie de 90 µm à 630 µm.

14. Procédé suivant la revendication 12 ou 13, caractérisé en ce que l'on mélange d'abord les composants A et B, on incorpore sous agitation l'agent antiblocage, l'additif réactif et la matière formant matrice, jusqu'à parvenir à l'homogénéité, pour autant que l'on soumette ce mélange à un traitement thermique à 25°C-180°C, de préférence, 100°C-120°C et, après l'addition de l'agent de réticulation, on fixe celui-ci sur la matrice à 25-180°C, de préférence, 50°C-80°C.

15. Procédé suivant la revendication 12 ou 13, caractérisé en ce que l'on mélange d'abord physiquement tous les composants et on entreprend ensuite un traitement thermique à 25°C-180°C, de préférence, 100°C à 120°C, afin de fixer l'agent de réticulation sur la matrice.

16. Procédé suivant les revendications 12 à 15, caractérisé en ce que l'on ajoute aux composants, avant le traitement thermique final, un solvant hydrophile, de préférence, en proportions de 1 à 10% en poids, par rapport au mélange des polymères.

17. Procédé suivant la revendication 16, caractérisé en ce que le solvant est l'eau ou un mélange d'eau et d'un solvant organique hydrophile, de préférence, un mélange d'eau et d'isopropanol.

18. Procédé de préparation d'une composition d'un matériau dépôt suivant la revendication 2, caractérisé en ce que le principe actif est absorbé, à partir de solutions dans l'eau ou contenant de l'eau de la composition suivant les revendications 1, 3 à 11 et on procède à un éventuel nouveau séchage, ou bien on l'ajoute, sous forme de solution ou de dispersion à la composition précitée, au cours de n'importe quelles étapes préalables du procédé de préparation.

19. Utilisation de la composition suivant les revendications 1 et 3 à 11 ou préparée suivant les revendications 12 à 18, sous forme de fibres, de films, de poudres ou de granulés, en vue de l'absorption de solutions et de dispersions contenant de l'eau ou de liquides (corporels) comme, par exemple, l'urine ou le sang, dans des produits chimicotechniques, comme, par exemple, des matériaux d'emballage, des récipients pour culture, en vue de l'amélioration ou de l'amendement de la terre et à titre d'enveloppement ou de gainage de câbles, dans des articles hygiéniques, comme, par exemple, des tampons ou des couches, ou des agents pour l'hygiène animale.

20. Utilisation de la composition suivant les revendications 2 à 1, ou préparée suivant la revendication 18, sous la forme de poudre ou sous la forme d'une dispersion dans des milieux hydrophobes, éventuellement en combinaison avec des stabilisateurs de dispersion ou en mélange à d'autres substances.

21. Articles pour l'hygiène (animale) contenant une composition suivant les revendications 1 à 11 ou préparée suivant les revendications 12 à 18.

22. Produits chimicotechniques contenant la composition suivant les revendications 1 à 11 ou préparée suivant les revendications 12 à 18.
